Europäisches Patentamt

European Patent Office

Office européen des brevets

(19)

(11) Veröffentlichungsnummer: **0 327 938**

**A1**

## (12) EUROPÄISCHE PATENTANMELDUNG

(21) Anmeldenummer: 89101686.7

(22) Anmeldetag: 01.02.89

(51) Int. Cl.⁴: **C07C 143/18 , C07C 139/00**

(30) Priorität: 09.02.88 DE 3803912

(43) Veröffentlichungstag der Anmeldung:
16.08.89 Patentblatt 89/33

(84) Benannte Vertragsstaaten:
AT BE CH DE ES FR GB GR IT LI NL SE

(71) Anmelder: Henkel Kommanditgesellschaft auf
Aktien
Postfach 1100 Henkelstrasse 67
D-4000 Düsseldorf 1(DE)

(72) Erfinder: Behler, Ansgar, Dr.
Siegfriedstrasse 80
D-4250 Bottrop(DE)
Erfinder: Piorr, Robert, Dr.
Kieselei 12
D-4030 Ratingen-Hösel(DE)

(54) Verfahren zur Herstellung von ungesättigten Fettsäureniedrigalkylester-sulfonaten.

(57) Ungesättigte Fettsäure-niedrigalkylester-sulfonate lassen sich durch Umsetzung eines Gemisches von ungesättigten und gesättigten Fettsäure-niedrigalkylestern mit gasförmigem Schwefeltrioxid bei einem molaren Einsatzverhältnis von Schwefeltrioxid zu ungesättigten Fettsäure-niedrigalkylestern von 1,0 bis 1,2 und bei Temperaturen von 15 bis 25 °C, wobei das erhaltene Produkt unmittelbar nach der Sulfonierung mit wäßrigen Basen unter Einhaltung eines pH-Wertes von mindestens 6 neutralisiert und hydrolysiert wird, herstellen. Die erhaltenen ungesättigten Fettsäure-niedrigalkylester-sulfonate weisen einen besonders niedrigen Gehalt an Fettsäuresulfonat-di-Na-salz auf.

EP 0 327 938 A1

# Verfahren zur Herstellung von ungesättigten Fettsäure-niedrigalkylester-sulfonaten

Die Erfindung betrifft ein Verfahren zur Herstellung von ungesättigten Fettsäure-niedrigalkylester-sulfonaten durch Umsetzung eines Gemisches von ungesättigten und gesättigten Fettsäure-niedrigalkylestern mit gasförmigen Schwefeltrioxid bei einem molaren Einsatzverhältnis von Schwefeltrioxid zu ungesättigten Fettsäure-niedrigalkylestern von 1,0 bis 1,2.

Ein Verfahren der vorstehend genannten Art ist aus der EP-A 0130 753 bekannt, dessen Ziel allerdings eine möglichst vollständige Sulfonierung der ungesättigten Fettsäureester ist. Zur Erzielung so hoher Sulfiergrade (bezogen auf die ungesättigten Fettsäureester) sind drastische Reaktionsbedingungen erforderlich, z.B. ein hoher Schwefeltrioxid-Überschuß (bezogen auf ungesättigte Anteile) sowie hohe Sulfierungstemperaturen. Zur Trennung der ungesättigten Fettsäure-niedrigalkylester-sulfonate von den nicht umgesetzten gesättigten Fettsäureestern nach der Neutralisation und Hydrolyse werden chemische Trennhilfsmittel wie Ethylenglykol, Propylenglykol und dergleichen eingesetzt; ähnliche Sulfier- und Trennverfahren sind weiterhin aus den DE-A 35 37 190 und 36 06 868 bekannt.

Bei der Umsetzung ungesättigter Fettsäure-niedrigalkylester mit Schwefeltrioxid erfolgt im Gegensatz zur Sulfonierung gesättigter Fettsäureester (alpha-Estersulfonierung) der Angriff des Schwefeltrioxids an der reaktiven C=C-Bindung und führt nach alkalischer Hydrolyse zu Estersulfonaten mit innenständiger Sulfonatgruppe. Dabei entstehen als Hauptprodukte Alken- und Hydroxyalkan-fettsäureestersulfonate. Als Nebenreaktion findet ein Einschub des Schwefeltrioxids in die Esterfunktion unter Bildung eines gemischten Anhydrids statt, welches formal aus sulfonierter ungesättigter Fettsäure und Methylschwefelsäure gebildet ist. Bei der alkalischen Hydrolyse zerfällt dieses gemischte Anhydrid in Methylsulfat und Fettsäuresulfonat-di-Na-salz (in folgenden als Disalz bezeichnet). Die Salze entstehen weiterhin auch bei der Hydrolyse der Esterbindung der Fettsäure-niedrigalkylester-sulfonate, d.h. bei der Hydrolyse der primären Sulfonierungsprodukte. Aus den verschiedensten Gründen ist die Anwesenheit größerer Mengen an Disalz in ungesättigten Fettsäure-niedrigalkylester-sulfonaten, deren Bildung niemals völlig unterdrückt werden kann, unerwünscht.

Die Erfindung ist auf ein Verfahren der eingangs genannten Art gerichtet, mit dem der Disalzgehalt in ungesättigten Fettsäure-niedrigalkylester-sulfonaten erheblich reduziert werden kann.

Diese Aufgabe wird durch ein Verfahren der eingangs genannten Art gelöst, bei dem man die Umsetzung mit Schwefeltrioxid bei Temperaturen von 15 bis 25 °C durchführt und das erhaltene Produkt unmittelbar nach der Sulfonierung mit wässerigen Basen unter Einhaltung eines pH-Wertes von mindestens 6 neutralisiert und hydrolysiert.

Die erhaltenen Verfahrensprodukte weisen die erwähnten niedrigen Disalzgehalte sowie eine überraschend geringe Verfärbung auf.

Die Sulfonierung der eingesetzten Gemische von ungesättigten und gesättigten Fettsäure-niedrigalkylestern mit gasförmigen Schwefeltrioxid kann in üblichen Sulfonierungsreaktoren, insbesondere vom Typ der Fallfilmreaktoren, erfolgen. Dabei wird Schwefeltrioxid mit Luft oder Stickstoff verdünnt eingesetzt, vorzugsweise in Form eines Gasgemisches mit ca. 1 - 10 Vol.-% Schwefeltrioxid.

Als für die Neutralisation bzw. Hydrolyse geeignete Basen eignen sich Oxide oder Hydroxide von Alkalimetallen wie Natrium, Kalium oder Lithium oder von Erdalkalimetallen wie Magnesium oder Calcium oder organische Basen wie Ammoniak, Ethanolamin, Diethanolamin oder Triethanolamin. Die Verwendung von wässriger Natronlauge ist bevorzugt.

Hervorzuheben ist, daß das verfahrensgemäß erhaltene Sulfonierungsprodukt unmittelbar der Neutralisation und Hydrolyse zugeführt wird; d.h. es ist - anders als bei üblichen Sulfonierungsverfahren für Ester gesättigter Fettsäuren - eine Nachreaktionszeit des Sulfonierungsproduktes vor der Hydrolyse und Neutralisation nicht vorgesehen.

Gemäß einer bevorzugten Ausführungsform des Verfahrens der Erfindung zur kontinuierlichen Sulfonierung und Neutralisation wird das Sulfonierungsprodukt neutralisiert und unter Zufuhr von wässrigen Basen zur Einhaltung eines pH-Wertes von 6 - 8 bei mindestens 70 °C hydrolysiert. Die obere Grenze der Hydrolysetemperatur ist durch den Siedepunkt des Wassers bestimmt; man kann jedoch auch die Hydrolyse in Druckreaktoren bei Temperaturen von mehr als 100 °C durchführen.

Die Einhaltung des hier angegebenen pH-Wertes ist kritisch; erfolgt z.B. lediglich die Neutralisation ohne weitere Basenzufuhr während der Hydrolyse, sinkt der pH-Wert des Reaktionssystems infolge von Umlagerungsreaktionen der Sulfonierungsprodukte rasch unter 6, wodurch die Disalzbildung gefördert wird. Die Disalzbildung wird weiterhin aus den vorgenannten Gründen auch gefördert, wenn der pH-Wert des Reaktionssystems für längere Zeit größer als 8 wird.

Gemäß einer weiteren vorteilhaften Ausführungsform der Erfindung zur diskontinuierlichen Durchführung gibt man das Sulfonierungsprodukt in eine vorgelegte wässrige Lösung der Base, wobei das molare Verhältnis von Base zu Schwefeltrioxid bzw. Sulfongruppen in dem Sulfonierungsprodukt mehr als 1:1 bis zu 1,3:1, insbesondere 1,1 bis 1,3, beträgt. Zwar erfolgt bei dieser Verfahrensvariante die Neutralisation und Hydrolyse zunächst bei pH-Werten von mehr als 8, dieser an sich ungünstige pH-Bereich wird jedoch bei dieser Verfahrensführung rasch unterschritten.

Normalerweise kann man auf eine nachträgliche Abtrennung der nicht umgesetzten gesättigten Fettsäure-niedrigalkylester aus dem erhaltenen Reaktionsgemisch verzichten. Wenn jedoch eine Abtrennung dieser nicht sulfonierten Produkte gewünscht ist, kann man gemäß einer weiteren vorteilhaften Ausführungsform der Erfindung das neutralisierte und hydrolysierte Sulfonierungsprodukt bei Temperaturen von 50 - 80, insbesondere von 50 - 70 °C, unter Ausschluß von chemischen Hilfsmitteln zur Phasentrennung in zwei Phasen trennen, wobei die einen Phase im wesentlichen die sulfonierten ungesättigten Fettsäure-niedrigalkylester-sulfonate und die andere Phase im wesentlichen die unsulfonierten gesättigten Fettsäuren-niedrig-alkylester enthält.

Gemäß einer weiteren vorteilhaften Ausführungsform der Erfindung setzt man in der Sulfonierung Gemische von gesättigten und ungesättigten $C_{16}$-$C_{22}$-Fettsäure-niedrigalkylestern ein, die mindestens 40, insbesondere 70 -90 Gew.-%, bezogen auf Gesamtgewicht der Fettsäureester, an einfach ungesättigten $C_{16}$-$C_{22}$-Fettsäureestern enthalten. Üblicherweise werden hier ölsäurereiche Fettsäureesterschnitte eingesetzt; es sind jedoch auch petrosen-und/oder erucasäurereiche Fettsäureesterschnitte einsetzbar.

Gemäß einer weiteren vorteilhaften Ausführungsform der Erfindung setzt man Gemische von Fettsäuremethylestern ein.

Die erfindungsgemäß hergestellten ungesättigten Fettsäure-niedrigalkylester-sulfonate können als oberflächenaktive Produkte eingesetzt werden.

Die Erfindung wird im folgenden anhand von bevorzugten Ausführungsbeispielen näher erläutert.

Beispiel 1.

Als Ausgangsmaterial wurde ein Fettsäuremethylester (Säurezahl 1,0; Verseifungszahl 192 - 197; Jodzahl 84 - 92) eingesetzt, dessen Fettsäureanteil folgende Zusammensetzung hatte (in Gewichtsprozent):

| $C_{12}$ gesättigt | 0 - 2 % |
|---|---|
| $C_{14}$ gesättigt | 2 - 5 % |
| $C_{15}$ gesättigt | 0 - 1 % |
| $C_{16}$ gesättigt | 4 - 6 % |
| $C_{17}$ gesättigt | 1 - 3 % |
| $C_{18}$ gesättigt | 1 - 3 % |
| $C_{20}$ gesättigt | 0 - 2 % |
| $C_{22}$ gesättigt | 0 - 2 % |
| $C_{16}$ einfach ungesättigt | 4 - 6 % |
| $C_{18}$ einfach ungesättigt | 63 - 73 % |
| $C_{18}$ zweifach ungesättigt | 7 - 12 % |

Die Sulfonierung wurde in einem Fallfilmreaktor aus Glas vorgenommen, der im wesentlichen aus einem von einem Heiz-und Kühlmantel umgebenen Rohr von 1100 mm Länge und 6 mm Innendurchmesser bestand. Der Reaktor war am Kopf mit einer Aufgabevorrichtung für das Esterausgangsmaterial und einem Gaseinleitungsrohr versehen. Gasförmiges Schwefeltrioxid, durch Erhitzen von Oleum erzeugt, wurde in Form einer Mischung mit Stickstoff (5 Vol.-% $SO_3$) eingesetzt.

Das Fettsäuremethylestergemisch wurde mit einer konstanten Geschwindigkeit von 550 g/h aufgegeben. Die Zufuhr des Schwefeltrioxid-Stickstoff-Gemisches wurde so eingestellt, daß das Molverhältnis von Schwefeltrioxid zu ungesättigten Fettsäuremethylestern 1,1 betrug. Mit Hilfe eines Wasserkreislaufs durch den Reaktormantel wurde die Reaktionstemperatur bei 15 °C gehalten.

Beim Verlassen der Reaktionszone wurde das Reaktionsgemisch in einem Becherglas aufgefangen, das mit der Elektrode eines pH-Meters und einem Rührer bestückt war. Das anfallende Reaktionsprodukt wurde durch Zugabe von 25-gewichtsprozentiger wäßriger Natriumhydroxidlösung sofort auf einen pH-Wert von 7 ± 1 neutralisiert.

Zur Hydrolyse wurde das neutralisierte Reaktionsprodukt anteilweise 6 Stunden lang auf 90 °C erhitzt. Durch Zugabe von 25-gewichtsprozentiger Natriumhydroxidlösung wurde dabei der pH-Wert der Lösung ständig auf 7 ± 1 gehalten. Anschließend wurde die auf Raumtemperatur abgekühlte wäßrige Phase mit Petrolether extrahiert.

In der klaren wäßrigen Phase wurde durch Titration nach Epton ein Waschaktivsubstanzgehalt (WAS-Gehalt) von 37,2 Gew.-% ermittelt, wobei 5,6 Gew.-% der WAS aus dem Dinatriumsalz der sulfonierten Fettsäuren bestanden.

Durch Abdampfen des Lösungsmittels aus dem Petroletherextrakt wurde der Anteil des nichtumgesetzten Ausgangsmaterials (im wesentlichen Methylester der gesättigten Fettsäuren) im Hydrolyseprodukt bestimmt. Dieser betrug 11,3 Gew.-%.

Beispiel 2.

Analog Beispiel 1 wurde der dort beschriebene Fettsäuremethylester bei 15° C und einem Molverhältnis von SO$_3$ zu ungesättigtem Ester von 1 : 1 sulfoniert.

Das neutralisierte Reaktionsprodukt wurde zur Hydrolyse 4 Stunden lang auf 90° C erhitzt.

In der erhaltenen wäßrigen Lösung wurde ein WAS-Gehalt von 28,1 Gew.-% festgestellt, wobei 14 Gew.-% der WAS aus dem Dinatriumsalz der sulfonierten Fettsäuren bestanden. Der Anteil des nicht umgesetzten Ausgangsmaterials in der wäßrigen Lösung betrug 10,0 Gew.-%.

Beispiel 3.

Analog Beispiel 1 wurde der dort beschriebene Fettsäuremethylester bei 15° C und einem Molverhältnis von SO$_3$ zu ungesättigtem Ester von 1,2 sulfoniert.

Das neutralisierte Reaktionsprodukt wurde zur Hydrolyse 4 Stunden lang auf 90° C erhitzt.

In der erhaltenen wäßrigen Lösung wurde ein WAS-Gehalt von 30,5 % festgestellt, wobei 14 Gew.-% der WAS aus dem Dinatriumsalz der sulfonierten Fettsäuren bestanden. Der Anteil des nicht umgesetzten Ausgangsmaterials in der wäßrigen Lösung betrug 9,0 Gew.-%.

Beispiel 4.

Der in Beispiel 1 beschriebene Fettsäuremethylester wurde wie in Beispiel 1 angegeben bei 15° C und einem Molverhältnis von SO$_3$ zu ungesättigtem Ester von 1,2 sulfoniert.

Das resultierende Sulfonierungsprodukt wurde in einem Becherglas aufgefangen und unter Rühren in eine solche Menge 25-gewichtsprozentiger Natriumhydroxidlösung eingetragen, daß das Molverhältnis von NaOH zu aufgenommenem SO$_3$ 1,2 betrug.

In der dabei erhaltenen klaren Lösung wurde ein WAS-Gehalt von 22 Gew.-% ermittelt. Eine Probe dieser Lösung wurde 8 Stunden lang auf 90° C erhitzt. Danach war der WAS-Gehalt unverändert 22 Gew.-%.

Beispiel 5.

Als Ausgangsmaterial diente ein Fettsäuremethylester (Säurezahl 1,0; Verseifungszahl 193 - 199; Jodzahl 45 - 53), dessen Fettsäureanteil folgende Zusammensetzung hatte (in Gewichtsprozent):

| | |
|---|---|
| $C_{12}$ gesättigt | 0 - 2 % |
| $C_{14}$ gesättigt | 2 - 7 % |
| $C_{15}$ gesättigt | 0 - 2 % |
| $C_{16}$ gesättigt | 25 - 33 % |
| $C_{17}$ gesättigt | 1 - 3 % |
| $C_{18}$ gesättigt | 15 - 19 % |
| $C_{20}$ gesättigt | 0 - 2 % |
| $C_{22}$ gesättigt | 0 - 2 % |
| $C_{16}$ einfach ungesättigt | 1 - 3 % |
| $C_{18}$ einfach ungesättigt | 44 - 48 % |
| $C_{18}$ zweifach ungesättigt | 2 - 4 % |

Dieser Fettsäuremethylester wurde wie in Beispiel 1 beschrieben bei 25°C mit Schwefeltrioxid im Molverhältnis $SO_3$ zu ungesättigtem Ester von 1,2 zur Reaktion gebracht. Das anfallende Reaktionsprodukt wurde sofort kontinuierlich auf einen pH-Wert von 7 ± 1 neutralisiert.

Das neutralisierte Reaktionsprodukt wurde zur Hydrolyse unter den in Beispiel 1 beschriebenen Bedingungen 4 Stunden lang auf 90°C erhitzt. Danach wurde das Gemisch auf 70°C abgekühlt, wobei Phasentrennung eintrat. Mit Hilfe eines Scheidetrichters wurde die wäßrige Phase von der öligen Phase getrennt. Anschließend wurde die auf Raumtemperatur abgekühlte wäßrige Phase mit Petrolether extrahiert.

In der wäßrigen Phase wurde durch Titration nach Epton ein WAS-Gehalt von 18,5 Gew.-% festgestellt.

Durch Abdampfen des Lösungsmittels aus dem Petroletherextrakt wurde der Anteil des nicht umgesetzten Ausgangsmaterials in der aus dem Hydrolyseprodukt abgetrennten wäßrigen Phase bestimmt. Dieser betrug 13,5 Gew.-%, bezogen auf WAS.


## Ansprüche

1. Verfahren zur Herstellung von ungesättigten Fettsäure-niedrigalkylester-sulfonaten durch Umsetzung eines Gemisches von ungesättigten und gesättigten Fettsäure-niedrigalkylestern mit gasförmigen Schwefeltrioxid bei einem molaren Einsatzverhältnis von Schwefeltrioxid zu ungesättigten Fettsäure-niedrigalkylestern von 1,0 bis 1,2, dadurch gekennzeichnet, daß man die Umsetzung bei Temperaturen von 15 bis 25°C durchführt und das erhaltene Produkt unmittelbar nach der Sulfonierung mit wässrigen Basen unter Einhaltung eines pH-Wertes von mindestens 6 neutralisiert und hydrolysiert.

2. Verfahren nach Anspruch 1, dadurch gekennzeichnet, daß man zur kontinuierlichen Sulfonierung und Neutralisation das Sulfonierungsprodukt neutralisiert und unter Zufuhr von wässrigen Basen zur Einhaltung eines pH-Wertes von 6 bis 8 bei mindestens 70°C hydrolysiert.

3. Verfahren nach Anspruch 1, dadurch gekennzeichnet, daß man zur diskontinuierlichen Durchführung das Sulfonierungsprodukt in eine vorgelegt wässrige Lösung der Base gibt, wobei das molare Verhältnis von Base zu Schwefeltrioxid bzw. Sulfongruppen in dem Sulfonierungsprodukt mehr als 1:1 bis zu 1,3:1, insbesondere 1,1 bis 1,3, beträgt.

4. Verfahren nach mindestens einem der Ansprüche 1 bis 3, dadurch gekennzeichnet, daß man das neutralisierte und hydrolysierte Sulfonierungsprodukt bei Temperaturen von 50 - 80, insbesondere von 50 - 70°C, unter Ausschluß von chemischen Hilfsmitteln zur Phasentrennung in zwei im wesentlichen ausschließlich ungesättigte sulfonierte Fettsäure-niedrigalkylester und unsulfonierte gesättigte Fettsäure-niedrigalkylester enthaltene Phasen trennt.

5. Verfahren nach mindestens einem Ansprüche 1 bis 4, dadurch gekennzeichnet, daß man Gemische von gesättigten und ungesättigten $C_{16}$-$C_{22}$-Fettsäure-niedrigalkylestern sulfoniert, die mindestens 40, insbesondere 70 - 90 Gew.-%, bezogen auf Gesamtgewicht der Fettsäureester, am einfach ungesättigten $C_{18}$-$C_{22}$-Fettsäureestern enthalten.

6. Verfahren nach mindestens einem Ansprüche 1 bis 5, dadurch gekennzeichnet, daß man Gemische von gesättigten und ungesättigten Fettsäuremethylestern einsetzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 969 375 (O. OKUMURA et al.) * Spalte 5, letzter Absatz, Spalte 6, erster Absatz * --- | 1 | C 07 C 143/18 C 07 C 139/00 |
| A | PATENT ABSTRACTS OF JAPAN Band 8, Nr. 241 (C-250)(1678), 6. November 1984; & JP - A - 59 122 454 (RAION K.K.) 14.07.1984 --- | 1 | |
| A | GB-A-2 174 089 (LION) * Anspruch 1 *; & DE - A - 36 06868 (Kat. D,A) ----- | 1 | |

| | RECHERCHIERTE SACHGEBIETE (Int. Cl.4) |
|---|---|
| | C 07 C 143/18 |

Der vorliegende Recherchenbericht wurde für alle Patentansprüche erstellt

| Recherchenort | Abschlußdatum der Recherche | Prüfer |
|---|---|---|
| BERLIN | 27-04-1989 | KAPTEYN H G |